Europäisches Patentamt

European Patent Office

Office européen des brevets

(11) Publication number : **0 283 244 B1**

## EUROPEAN PATENT SPECIFICATION

(45) Date of publication of patent specification :
**12.02.92 Bulletin 92/07**

(21) Application number : **88302244.4**

(22) Date of filing : **15.03.88**

(51) Int. Cl.⁵ : **C12N 9/02,** C12N 15/53, A61K 37/50, C12N 1/21

(54) **Superoxide dismutase polymers.**

(30) Priority : **16.03.87 US 26143**

(43) Date of publication of application :
**21.09.88 Bulletin 88/38**

(45) Publication of the grant of the patent :
**12.02.92 Bulletin 92/07**

(84) Designated Contracting States :
**AT BE CH DE ES FR GB GR IT LI LU NL SE**

(56) References cited :
**EP-A- 0 138 111
EP-A- 0 180 964
EP-A- 0 196 056
Proceedings of the National Academy of Sciences of the United States of America, vol. 77, n 2, February 1980, Baltimore, USA. W.F. Petrone et al "Free radicals and inflammation : superoxide-dependant activation of a neutrophil chemotactic factor in plasma", pages 1159-1163**

(56) References cited :
**Analytical biochemistry, vol. 131, 1983, New York. C.O. Beauchamp et al "A new procedure for the synthesis of polyethylene glycol-protein adducts. Effects on function, receptor recognition, and clearance of superoxide dismutase, lactoferrin, and x2-macroglobulin", pages 25-33
Agents and actions, vol. 10, n 3, 1980, Basel, Schweiz. K. Wong et al "Enhanced antiinflammatory effect and reduced immunogenicity of bovine liver superoxide dismutase by conjugation with homologous albumia", pages 231-239.
Federation proceedings, vol. 44, n 8, May 1985, Washington DC. B.A. Freeman et al "Modulation of oxidant lung injury by using liposome-entrapped superoxide dismutase and catalase", pages 2591-2595**

(73) Proprietor : **CHIRON CORPORATION
4560 Horton Street
Emeryville, California 94608 (US)**

(72) Inventor : **Hallewell, Robert A.
27 Priest Street
San Fancisco, CA 94109 (US)**
Inventor : **Mullenbach, Guy
339 63rd Street
Oakland, CA 94618 (US)**

(74) Representative : **Goldin, Douglas Michael et al
J.A. KEMP & CO. 14, South Square Gray's Inn
London WC1R 5EU (GB)**

## Description

<u>Technical Field</u>

This invention is in the fields of protein chemistry and genetic engineering. More particularly, it relates to polymers of Cu/Zn superoxide dismutase (hereinafter referred to as SOD) that exhibit longer halflives <u>in vivo</u> than native SOD.

<u>Background</u>

SOD is a member of a family superoxide dismutases which, because of their ability to catalyze the destruction of superoxide ions, renders them useful in a variety of therapeutic settings such as in reducing perfusion injury and in treating inflammation. The amino acid sequence of human SOD (hSOD) is described in Jabusch et al, Biochemistry (1980) 19:2310-2316. The cloning and sequencing of hSOD cDNA and the production of hSOD in bacteria and yeast are described in EPA 84111416.8 (published 24 April 1985 under number 0 138 111). hSOD is normally a homodimer of two chains bound together by hydrophobic interaction. The homodimer has a molecular weight of approximately 32 kd.

SOD is rapidly removed from circulation by the kidney. Rat studies indicate its half-life in serum is on the order of seven minutes. The limited circulatory life of injected SOD may severely limit clinical approaches to SOD pharmacology and the effectiveness of treatment. In order to eliminate SOD's susceptibility to removal by the kidney, prior workers have conjugated SOD with macromolecules such as Ficoll (PNAS, USA (1980) 77:1159-1163), polyethylene glycol (Res. Commun. in Chem. Path. & Pharmacol. (1980) 29:113-120; Pharmacol. Res. Commun. (1982) 14:113-119; Anal. Biochem. (1983) 131:25-30; and J. Pharm. & Pharmacol. (1983) 35:757763), and human serum albumin (Agents Actions (1980) 10:231-240). SOD has been entrapped in liposomes for the same purpose (J. Clin. Invest. (1984) 73:87-95; Fed. Proc. (1985) 44:2591-2595).

An object of the present invention is to provide SOD in a novel polymeric form that has an extended circulatory life and retains the activity of SOD. This polymeric SOD may be made using recombinant DNA techniques. Its manufacture does not involve any chemical derivatization such as that described above. A preferred embodiment of this polymeric SOD comprises SOD monomers covalently linked to each other via a polypeptide that has an amino acid sequence, such as the sequence of the hinge region of immunoglobulin, which naturally acts as a means of coupling functional domains of biologically active proteins in a manner that allows them to fold and function independently. In this regard, <u>Staphylococcus</u> <u>aureus</u> nuclease and the c-myc oncogene have both been attached to a murine immunoglobulin heavy chain via the hinge region of the immunoglobulin to produce functional antibody-enzyme complexes (Nature (1984) 312:604-608).

<u>Disclosure of the Invention</u>

The invention provides novel SOD polymers formed from SOD units of at least two SOD monomers covalently coupled carboxy terminus to amino terminus by a polypeptide spacer of at least three amino acids.

Parenteral pharmaceutical compositions containing these new SOD polymers and therapeutic methods that employ them are other aspects of the invention.

The SOD units are also novel and are a part of the invention.

The invention also encompasses the biological materials (DNA encoding the SOD polymers, expression vectors containing such DNA, and host microorganisms or cells capable of expressing such DNA) and the methods involved in making the SOD polymers.

<u>Brief Description of the Drawings</u>

Figure 1 shows the sequence of a DNA fragment that encodes hSOD monomer and the amino acid sequence of hSOD monomer.

Figure 2 is a flow chart for the construction of the plasmid pCl/1SODHA1 described in the examples.

Figure 3 is a flow chart for the construction of the plasmid pCl/1SODHA1-met described in the examples.

Figure 4 is a photograph of an SDS gel analysis of the protein identified as SODHA1 in the examples, through its purification.

Figure 5 is a photograph of an agarose gel analysis of the protein identified as SODHA1 in the examples, through its purification.

Figure 6 is an absorbance tracing of eluate from a gel filtration of the purified protein identified as SODHA1 in the examples.

Figure 7 is a photograph of an activity-stained PAGE analysis of the fractions from the gel filtration shown in Figure 6.

Modes for Carrying Out the Invention

A. Definitions

The term "SOD monomer" intends the monomer (single chain) that forms the normal SOD homodimer.

The term "SOD unit" intends a molecule composed of at least two SOD monomers covalently coupled head-to-tail by a polypeptide spacer. The monomers may be the same or different.

The term "SOD polymer" intends a composition composed of at least two SOD units bound together by hydrophobic interaction.

The term "SOD intends a polypeptide having the amino acid sequence of native Cu/Zn superoxide dismutase and analogs or muteins thereof having substantially homologous amino acid sequences thereto which may differ in one or more amino acid substitutions, additions or deletions but still retain the enzymatic activity of native Cu/Zn superoxide dismutase. Examples of such analogs are those described in commonly owned copending EPO application Serial No. 88300294.1, filed 14 January 1988. The term encompasses SOD of various mammalian species. hSOD is preferred for use in humans. The term "substantially homologous" intends at least about 90% identity more usually at least about 95% identity, in amino acid sequence.

The number of SOD units in the polymers of the invention will usually be between about 2 and about 10. Preferably the number of units is between 2 and 4 and most preferably the number of units is 2. The polymers are generally linear with the monomers covalently coupled carboxy terminus to amino terminus via the polypeptide spacer. The spacer will normally be 3 to 1000 amino acids in length, preferably between about 10 and about 100 amino acids in length. Its amino acid composition and sequence is such that it is substantially nonimmunogenic (i.e., does not cause an unacceptable immune response), and substantially insensitive to proteases (i.e., is not readily digested by the proteases that occur in circulation). The spacer will typically be rich in proline and the proline content of the spacer will normally be in the range of 20 to 50 number percent, preferably 25 to 45 number percent. The sequence also preferably lacks cysteines (to avoid the likelihood of cross-linking) and is preferably substantially nonhydrophobic. The spacer's hydrophobicity may be determined by summing the hydrophobicities of the individual amino acids (measured by partition coefficient tests) of which it is composed. A substantially nonhydrophobic sequence will measure neutral or hydrophilic.

Preferred spacers have amino acid sequences that correspond to sequences, such as those of the hinge region of immunoglobulins, that act as connector domains that link functional domains of biologically active endogenous (naturally-occurring in the individual to whom the SOD polymer is to be administered) proteins in a manner that allows them to assume an appropriate tertiary structure and function independently.

Particularly preferred subgroups of SOD polymers are those represented by the formulas:

(SOD monomer-IgA-SOD monomer)$_x$ or

(SOD monomer -IgA-SOD monomer-Iga-SOD monomer)$_x$

where SOD monomer is as defined previously, IgA represents a 10 to 100 amino acid long segment of IgA hinge region, and x is an integer in the range of 2 and 4, inclusive. Preferably x is 2.

The SOD polymers of this invention are made by recombinant DNA techniques. Briefly, DNA encoding the SOD unit is made by ligating DNA fragments encoding the SOD monomer and spacer together. The DNA may be genomic, cDNA or synthetic DNA. The DNA encoding the polymer may be inserted into suitable prokaryotic or eukaryotic replicons (a genetic element such as a plasmid, a chromosome, or a virus that behaves as an autonomous unit of polynucleotide replication within a cell), the resulting expression vectors incorporated into suitable host organisms or cells, the recombinant organism or cell grown under conditions that result in expression of the DNA, and the resulting SOD polymer isolated from the host or, if secreted, from the growth medium using the same techniques as are described in EPA 841111416.8 to produce recombinant hSOD. The disclosure of that EPA is incorporated herein by reference.

In creating an expression vector, the DNA encoding the SOD unit is located in the vector with the appropriate control DNA sequences, which include a promoter, a ribosomal binding site, and transcriptional and translation stop codons. The positioning and orientation of the coding sequence with respect to the control sequences is such that the coding sequence is transcribed under the "control" of the control sequences-i.e., the promoter will control the transcription of the mRNA derived from the coding sequence, the ribosomes will bind at the ribosomal binding site to begin the translational process, and the stop codon used to terminate translation will be upstream from the transcriptional termination codon. In addition to control sequences, it may be desirable to add regulatory sequences which allow for regulation of the expression of the SOD polymer gene relative to the growth of the host cell.

The SOD polymers of the invention may be used for the same purposes as SOD. Because they have longer half-lives in circulation, the SOD polymers are particularly useful in pharmaceutical formulations intended for parenteral (intramuscular, intravenous, intraperitoneal, etc.) administration. The SOD polymers may be used in human or veterinary medicine to treat (i.e. cure, alleviate or prevent) a variety of conditions. They are useful as antiinflammatory agents, chemopreventive agents to prevent oncogenesis and tumor promotion, protective agents to reduce cytotoxic and cardiotoxic effects or anticancer drugs or protect ischemic tissue. Like native SOD, the SOD polymers catalyze the reduction of superoxide radicals to hydroperoxide and molecular oxygen and may thus be used to reduce perfusion injury following ischemia, prolong the viability of excised isolated organ transplants, reduce injury on reperfusion following organ transplant or spinal cord ischemia, reduce cardiac infarct size, reduce spinal cord injury and treat bronchial pulmonary dysplasia.

When used to treat tissues in vitro the polymer will be added to the perfusion or culture medium. When used in vivo, the SOD polymer may be administered neat or admixed in effective amounts with pharmaceutically acceptable injectable vehicles. Preferably the SOD polymer is conveniently stored lyophilized with sugar, usually sucrose, usually in a ratio of 1:2 w/w. The lyophilized SOD polymer is conveniently reconstituted in a suitable diluent for the particular application. For example, to treat inflammatory joint disease the SOD polymer may be reconstituted in physiologic saline in a volume convenient for intraarticular administration.

The dose of SOD polymer administered to an individual will depend upon the nature of the individual being treated, the mode of treatment and the condition being treated. In general the amount administered must be sufficient to provide an enzymatically effective amount of the SOD polymer at the desired site of treatment. In this regard when the SOD polymer is administered systemically, larger doses will typically be required than when the SOD polymer is administered locally at the site that requires treatment. By way of example, human patients having inflammatory joint disease are treated by a weekly intraarticular injection into a joint afflicted with the disease of a solution having hSOD mutein in a suitable diluent in an amount effective to reduce inflammation, usually 1 to 10 mg, more usually 2 to 6 mg. The injection are given weekly for a period of time sufficient to reduce inflammation, usually for 2 to 8 weeks, more usually for 4 to 6 weeks. Because the articular capsule limits leakage of the high molecular weight compound each afflicted joint should be treated with the required dosage. When used to minimize post-ischemic tissue damage the human patient is administered 10 mg to 1,000 mg, more usually 50 mg to 500 mg of SOD polymer in a suitable diluent during the ischemic reaction. When the patient suffers ischemia due to a disease the solution is administered intravenously or intraarterially as a bolus dosage or a continuous infusion. In such situations the SOD polymer may be administered in conjunction with fibrinolytic agents such as urokinase, streptokinase or tissue plasminogen activator (TPA). When ischemic damage is due to a surgical procedure, SOD polymer is administered during surgery. This application finds particular use in organ transplant surgery where SOD is preferably administered prior to reirrigation of the organ and is also useful in any other surgery where bloodflow to an organ is interrupted, such as open heart surgery.

The following examples further illustrate the invention. These examples are not intended to limit the invention in any manner.

Construction of Yeast Plasmid pCI/1SODHA1

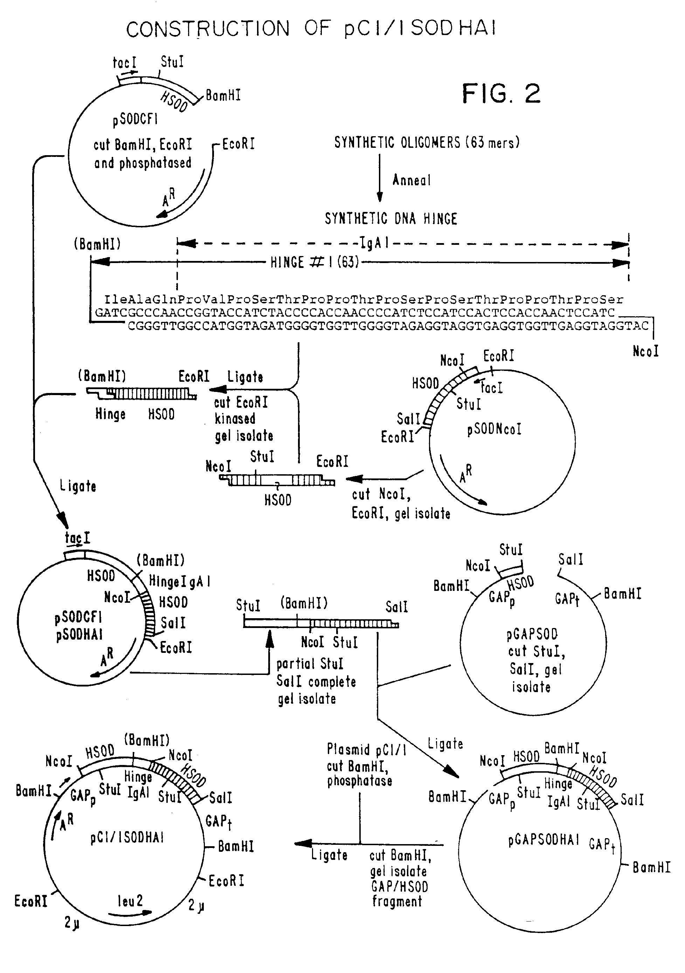

The construction of plasmid pCI/1SODHA1 is depicted in Figure 2.

DNA oligomers (63 mers) were synthesized and annealed to form a fragment encoding the human IgAl hinge region beginning at amino acid residue 226 to avoid the Cys 225 residue (See Kabat, E.A., et al., In Sequences of Proteins of Immunological Interest (1983) U.S. Dept. of Health & Human Resources, PHS, NIH, p.179, for sequence of IgA heavy chain) with BamHI and NcoI sites at its ends. The fragment had the sequence shown below.

This fragment was used to make plasmid pSODCF1SODHA1 encoding bacterial expression of spacer-linked hSOD monomers as follows.

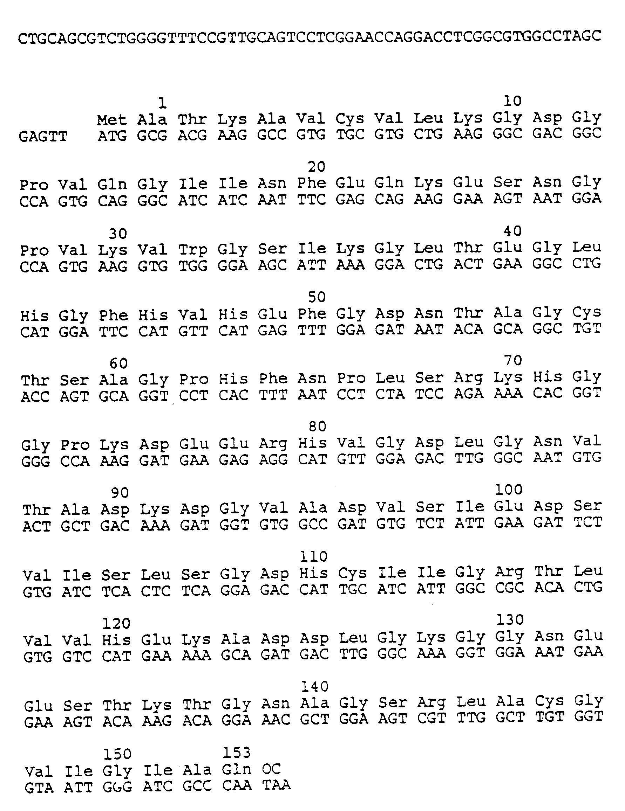

The plasmid phSOD (designated pSODNcol in Figure 2 and described in commonly owned EPA 84111416.8, the disclosure of which, as it relates thereto, is incorporated herein by reference) was cut with NcoI and EcoRI and a NcoI-EcoRI fragment containing the hSOD DNA shown in Figure 1 was gel isolated from the digest. This fragment was ligated to the synthetic DNA fragment encoding the IgA hinge region. The ligation product was kinased, cut with EcoRI and the BamHI-EcoRi fragment encoding the IgA hinge fused to hSOD was gel isolated.

The IgA hinge-hSOD fragment was then inserted into plasmid pSODCF1, which had been cut with BamHI and EcoRI and phosphatased to form plasmid pSODCF1SODHA1.

pSODCF1 was derived from plasmid pSODX8 (described in Hallewell, et al, Nucleic Acids Res. (1985) 13:2017-2034) as follows. Plasmid pSODX8 was cut with StuI and SalI. An approximately 400 bp fragment was gel isolated and partially digested with Sau3A and a 327 bp hSOD fragment was recovered. Plasmid pSODX16 (described in Nucleic Acids Res. (1985) 13:2017-2034) was cut with StuI and BamHI and the large vector fragment was gel isolated from the digest. This vector fragment was ligated to the 327 bp hSOD fragment to produce plasmid pSODCF1.

pSODCF1SODHA1 was subjected to partial digestion with StuI and complete digestion with SalI, and a 560 bp StuI-SalI fragment was gel isolated from the digest.

The plasmid pGAPSOD was cut with StuI and SalI and vector DNA was gel purified. pGAPSOD is a yeast promoter vector containing the hSOD gene under the regulation of the glyceraldehyde-3-phosphate dehydrogenate (GAP) promoter and terminator. Details of its composition and construction are described in commonly owned EPA 84111416.8, the disclosure of which, as it relates to this plasmid, is incorporated herein by reference. (The plasmid is designated pPGAPSOD in EPA 84111416.8.)

The 560 bp StuI-SalI fragment from pSODCF1SODHA1 was ligated with the vector DNA from pGAPSOD to form plasmid pGAPSODHA1. pGAPSODHA1 was then cut with BamHI and the resulting BamHI cassette (GAP/hSOD fragment) was gel isolated. The cassette was then subcloned in the expression orientation in the yeast shuttle vector pCI/1, which had been digested with BamHI. Plasmid pCI/1 is a derivative of pJDB219 (Beggs, Nature (1978) 275:104) in which the region corresponding to bacterial plasmid pMB9 in pJDB219 is replaced by pBR322.

Construction of Yeast Plasmid pCI/1SODHA1-met

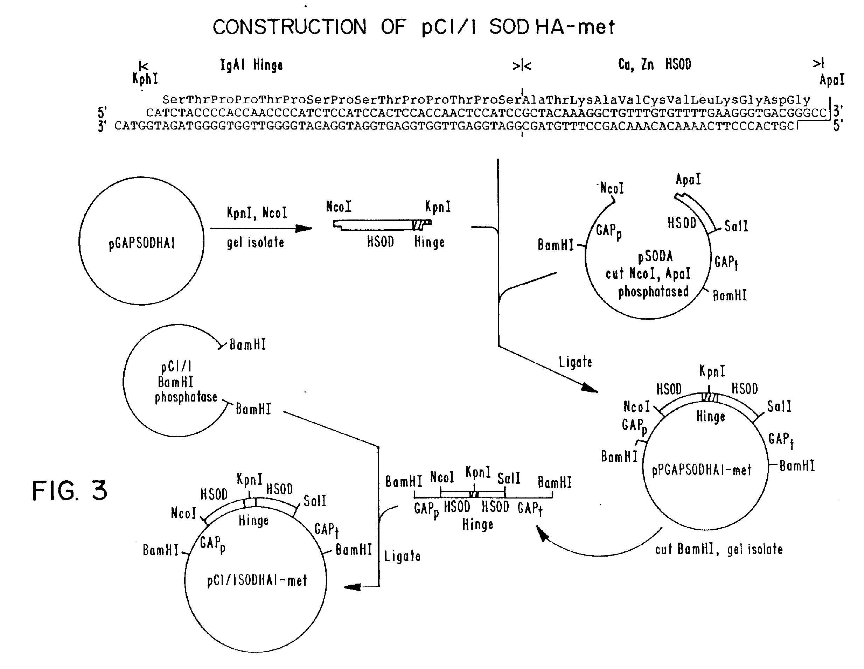

The construction of pCI/1SODHA1-met is shown in Figure 3. This plasmid was designed for expression of an hSOD polymer identical to the hSOD expressed by pCI/1SODHA1, except for the elimination of the methionine at the C-terminus of the IgAI sequence of pCI/1SODHA1. In this regard, methionine is normally removed from the N-terminus of hSOD and it was felt the elimination of this met might lessen the likelihood of immunogenicity.

The DNA fragment shown below encoding portions of the IgAI hinge and hSOD was synthesized.

```
        K                    IgAl Hinge         >|<      Cu,Zn    HSOD
      KpnI                                                                              ApaI
        ...SerThrProProThrProSerProSerThrProProThrProSerAlaThrLysAlaValCysValLeuLysGlyAspGlyPro|
   5' CATCTACCCCACCACCCCATCTCCATCCACTCCACCAACTCCATCGCCTACAAAGGCTGTTTGTGTTTTGAAAGGGTGACGGGCG 3'
   3' CATGGTAGATGGGGTGGTTGGGGTAGAGGTAGGTGAGGTGGTTGAAGGTAGGCGATGTTTCCGACAAACACAAAACTTCCCACTGC 5'
```

pGAPSODHA1 was cut with KpnI and NcoI and the small hSOD-IgAl hinge fragment was gel purified from the digest.

The plasmid pSODA, a yeast promoter vector containing an hSOD gene in which the Cys6 codon is replaced with an Ala-encoding triplet described in commonly owned EPA 88300294.1, the disclosure of which is incorporated herein by reference to the extent it relates to said plasmid, was cut with NcoI and ApaI and phosphatased. (pSODA is designated pYSODA in EPA 88300294.1.) The resulting vector DNA was ligated with the above-described synthetic DNA fragment and the KpnI-NcoI fragment from pGAPSODHA1 to form plasmid pGAPSODHA1-met. A BamHI expression cassette was isolated from the plasmid by digestion with BamHI and gel purification and subcloned into pCI/1 as above to form the yeast expression vector pCI/1SODHA1-met.

Preparation of Recombinant Yeast Strains

Plasmids pCI/1SODHA1 and pCI/1SODHA1-met were transformed into S. cerevisiae strain P017 (Mat a, leu 2-04, cir°), as described in PNAS USA (1978) 75:1929-1933, selecting on agar medium lacking leucine, to produce recombinant strains that produce an hSOD unit composed of two hSOD monomers joined covalently by the 23 amino acid IgAl hinge fragment shown above (designated SODHA1) and a similar unit lacking a met residue at the N-terminus of the second hSOD unit (designated SODHA1-met).

Yeast strain P017 was obtained by isolating a spontaneous revertant strain 2150-2-3 (Mat a, ade 1, leu 2-04, cir°). To isolate the revertant P017, 2150-2-3 yeast cells were grown in YEPD, washed in medium without adenine and about 6 x 10⁸ cells were plated onto six adenine minus (ade-) plates. Four candidate revertants were obtained and they were restreaked onto adeplates. The revertants were tested for other genetic markers by streaking on plates without uracyl (ura-), plates with no leucine (leu-), and minimal plates plus leucine. Growth was observed on uraand minimal plus leu plates; no growth on leuplates. Revertants were crossed with strain AB103.1 (Mat α pep 4-3, leu 2-3, leu 2-112, ura 3-52, his 4-580) to determine if the reversion was due to extragenic suppression. Based on tetrad analysis, none of the four independent ade+ revertants were due to extragenic suppressor. Based on good growth and high spore viability, one of the revertants was selected and named P017.

Expression and Purification of SODHA1 from Yeast

A 10 l culture of yeast strain P017 transformed with pCl/1SODHA1 was grown to stationary phase in YEPD medium (Sherman, F., Fink, G.R., and Hicks, J.B., Methods of Yeast Genetics (1982), Cold Spring Harbor, New York) containing 3 mM CuSO₄ by inoculating the culture with 500 ml starter culture in minimal glucose medium lacking leucine.

One hundred seventy-three g of yeast cells expressing SODHA1 were lysed in 20 mM Tris-Cl, pH 8. After centrifugation, 900 ml of extract were obtained, the pH adjusted to 8.0 with NaOH, and stored at -20°C. Eight hundred twenty ml were heated for 2 hr at 65°C, centrifuged, and the supernatant diluted with 8 vol 20 mM Tris-HCl, pH 8, to bring the conductivity down to 1 mmho. This was loaded onto a 400 ml column of DEAE Sepharose® equilibrated with the same buffer. The column was washed extensively with 20 mM Tris-HCl, pH 8, and the SODHA1 eluted with 20 mM Tris-HCl, pH 9, 0.5 M NaCl. The eluate was sterile filtered and stored at 4°C. Details of this purification are reported in Table 1 below.

EP 0 283 244 B1

Table 1

Purification of SODHA1

| | Volume (ml) | Protein (mg/ml) | Total Protein (g) | Units (ml) | Total Units | Specific Activity* (Units/g) | Fold Purification | Yield (%) |
|---|---|---|---|---|---|---|---|---|
| Extract | 820 | 13 | 10.7 | 10 | 8200 | 766 | 1.0 | 100 |
| Heated Extract | 720 | 6 | 4.3 | 11 | 7920 | 1842 | 2.25 | 97 |
| DEAE Eluate | 400 | 9 | 3.6 | 17 | 6800 | 1889 | 2.40 | 83 |

*Measured by pyrogallol method, Eur. J. Biochem (1974) 47:469-474.

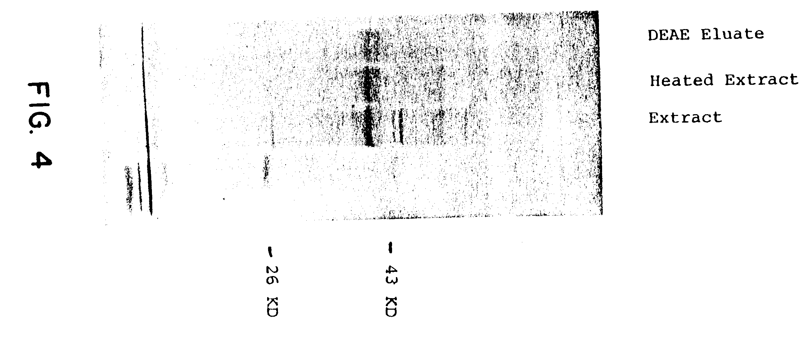

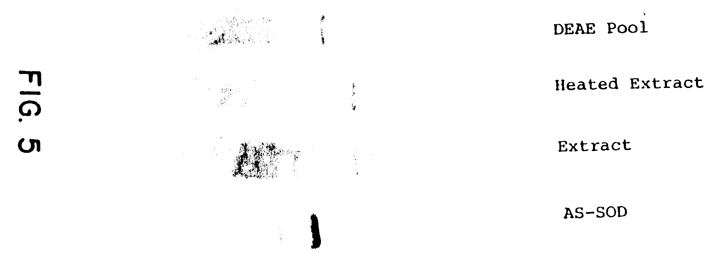

As reported in Table 1, the yield was 83% overall, with a 2.4-fold publication. Since the SODHA1 is nearly pure, this indicates that it originally represented about 40% of the total protein found in the extract. Both the heating step and column gave good yields, 97% and 83%, respectively. The heating step was responsible for most of the purification (see figures 4 and 5), but the column was valuable in removing nonproteinaceous contaminants such as lipid, carbohydrate, and nucleic acid.

An SDS gel (Coomassie Blue stain) of SODHA1 in various stages of purification is shown in Figure 4. Again, it is immediately evident that most of the purification occurs during the heating step. The figure also shows that there is a shift in mobility during the purification, resulting in a product that has 3 bands, the fastest migrating of which comigrates with the SODHA1 in the crude extract. This shift towards apparently higher molecular weight forms occurred both during the heating step and the ion-exchange chromatography. the cause of this alteration in the protein is unknown, but it does not seem to have very much effect on its function, since the SODHA1 has retained nearly all its activity after the purification. In fact, the purified SODHA1 has approximately the same specific activity as recombinant native hSOD using the pyrogallol assay.

As a further test for alterations in SODHA1 during its purification, aliquots were subjected to agarose gel electrophoresis (Figure 5). The procedure used Corning Universal 1% agarose gel equilibrated with 5 mM Tris, 30 mM EDTA. The gel was electrophoresed for 20 min at 250 V and stained with Amido Black. Lane 1 contains crude extract, lane 2 heated extract, and lane 3 DEAE column eluate. Clearly, the SODHA1 actually exists as a mixture of species separable on this gel system. After heating, and after elution off the column, there are minor changes in the gel pattern. The identities of these species are unknown, but it appears that no major changes have occurred in the distribution of SODHA1 forms.

Characterization of the Purified SODHA1

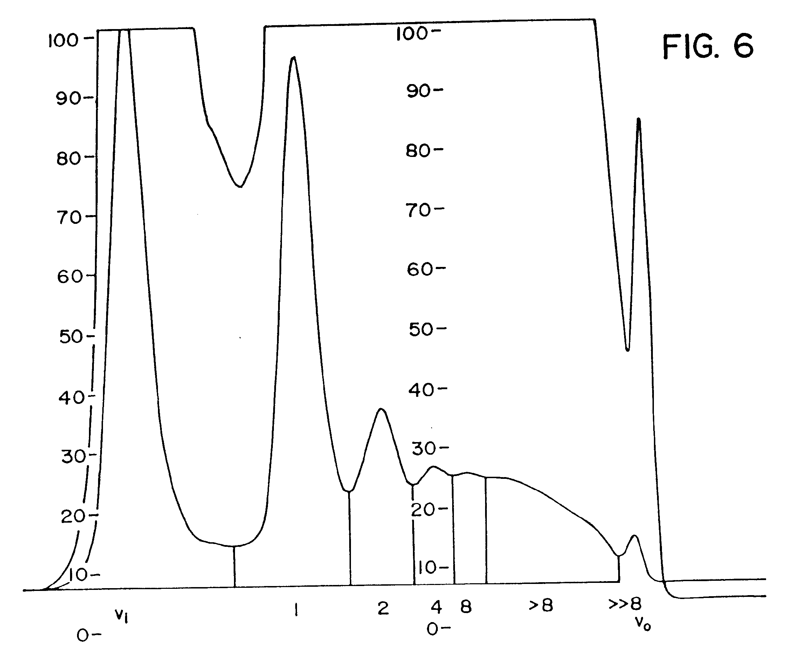

In addition to the experiments described above characterizing the purified SODHA1, the size distribution of species was analyzed by gel filtration chromatography. Here, aliquots of crude extract and DEAE eluate were chromatographed on a calibrated AcA34 column, yielding essentially identical results (the crude sample contained more UV-absorbing material in the void and included volumes; this was not, however, SODHA1). Figure 6 shows the $OD_{280}$ absorbance tracing of material eluted off the column loaded with purified SODHA1. Two tracings are shown: The more sensitive one is 0.2 absorbance 280 units full scale, the other 2.0 absorbance units. The void volume (Vo) and included volume (Vi) are marked. The peaks are labeled with the probable number of covalent dimers per molecule in that peak (see Table 2).

Except for some UV-absorbing contaminating material eluting in the included volume, the absorbance tracing directly reflects the presence of SODHA1 (as assayed by SDS gel electrophoresis). The size distribution is presented in Table 2 below, as well as an interpretation of the subunit structures of the species, e.g., the 32 kd peak is almost certainly a single molecule of SODHA1. The 75 kd is likely to be a dimer of SODHA1, and the 125 kd, a trimer or possibly a tetramer of SODHA1. If these assignments are correct, nearly half of the protein is present as SODHA1 of nearly the same size as native SOD. One-sixth is approximately twice the size of native SOD, one-twelfth is approximately three times its size, etc. In summary, about half of the total mass is as least approximately twice the size of native SOD.

## Table 2

### Size Distribution of SODHA1 Multimers

| Apparent Molecular Weight (kd) | Apparent Number of Units per Molecule | % of Total |
|---|---|---|
| 32 | 1 | 46.4 |
| 85 | 2 | 16.9 |
| 155 | 3 or 4 | 8.2 |
| 222 | 8 | 7.5 |
| 223-750 | >8 | 19.0 |
| 750 | >>8 | 2.0 |

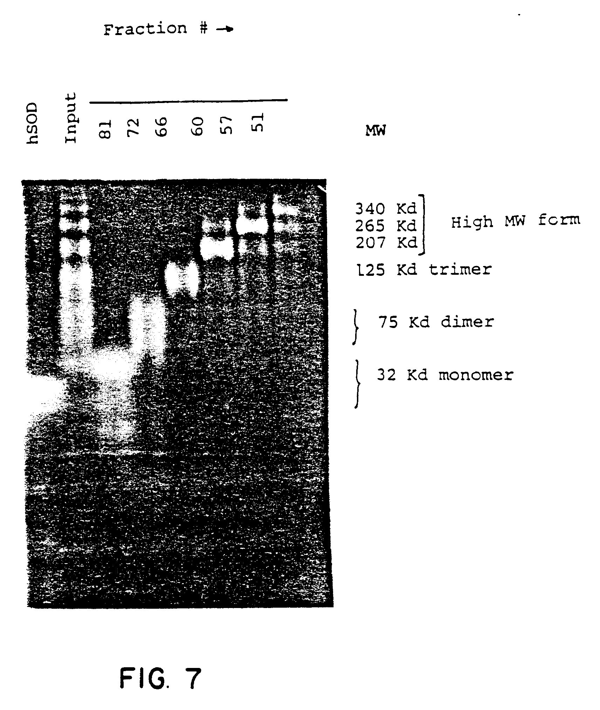

Interestingly, all these species are active. When aliquots from the sizing column, loaded with crude extract here, were subjected to native polyacrylamide gel electrophoresis and stained for activity (hSOD was

EP 0 283 244 B1

electrophoresed as a standard, and the column input is in the adjacent line). The results are shown in Figure 7. All the species resolvable on the gel were seen to be active (fractions greater than 51 not shown here).

In conclusion, 3.6 g of SODHA1 has been purified by a combination of heating and ion-exchange chromatography. The SODHA1 exists as a population of active species, approximately half of which by mass is a monomeric form of SODHA1, with a distribution of various higher molecular weight species including some larger than 750 kd.

## Stability of SODHA1 in Rat Plasma

Anesthetized rats (ca. 300 g in weight) were injected in the tail vein with 2,000 units (ca. 5 mg) of native hSOD, SODHA1, purified ca. 75 kd dimer, purified ca. 125 kd trimer, and higher molecular weight multimers (HMW-1). Serum samples (0.5 ml) were prepared from tail vein blood at 1, 5, 15, 30, 60, 120, 240 min and 24 hr. These samples were assayed for superoxide dismutase activity using the pyrogallol method. The results are shown in Table 3.

## Table 3

### Blood Plasma Retention of Multimeric Forms of SODHA1 in Rats (in SOD U/ml of plasma)

| Sample Time | Native Cu,Zn SOD | Monomer SODHA1 | Dimer SODHA1 | Trimer SODHA1 | HMW-1 SODHA1 | Total SODHA1 |
|---|---|---|---|---|---|---|
| 0' | 5.2 | 5.8 | 5.3 | 6.3 | 5.8 | 5.0 |
| 1' | 350 | 358 | 368 | 253 | 281 | 281 |
| 5' | 240 | 242 | 357 | 253 | 263 | 231 |
| 15' | 131 | 136 | 316 | 232 | 250 | 170 |
| 30' | 51 | 55 | 258 | 205 | 237 | 150 |
| 60' | 22 | 26 | 226 | 163 | 190 | 128 |
| 120' | 14 | 13 | 195 | 102 | 158 | 80 |
| 240' | 10 | 11 | 111 | 90 | 118 | 60 |
| 24h | 8 | – | – | – | 38 | 20 |

These data indicate that native hSOD and the monomeric form of SODHA1 are both very rapidly removed from plasma with a half-life of approximately 7 min (taking 1 min as the 100% value). In contrast, the larger forms of hSOD (SODHA1 dimer, trimer, HMW-1, and total SODHA1) with molecular weights of greater than 75,000 have much longer half-lives on the order of 60-120 min.

Modifications of the above-described modes for carrying out the invention that are obvious to those of skill in the fields of biochemistry, genetic engineering, pharmacology, and related fields are intended to be within the scope of the following claims.

## Claims

**Claims for the following Contracting States : AT, BE, CH, DE, FR, GB, IT,LI, LU, NL, SE**

1. A Cu/Zn superoxide dismutase (SOD) polymer of SOD units of at least two SOD monomers covalently coupled, carboxy terminus to amino terminus, to each other by a polypeptide spacer of at least three amino acids.

2. The polymer of claim 1 wherein the SOD is human SOD (hSOD).

3. The polymer of claim 2 wherein each hSOD unit has two hSOD monomers.

4. The polymer of claim 3 wherein the polymer contains 2 to 10 hSOD units.

5. The polymer of claim 3 wherein the polymer contains 2 to 4 hSOD units.

6. The polymer of claim 2 wherein the polymer contains 2 hSOD units.

7. The polymer of claim 1, 2, 3, 4, 5 or 6 wherein the spacer is 3 to 1000 amino acids in length, substantially nonimmunogenic, and substantially insensitive to proteases.

8. The polymer of claim 1, 2, 3, 4, 5 or 6 wherein the spacer is 10 to 100 amino acids in length, substantially nonimmunogenic, and substantially insensitive to proteases.

9. The polymer of claim 7 or 8 wherein the spacer lacks cysteine residues and is substantially nonhydrophobic.

10. The polymer of claim 2 wherein each hSOD unit has three hSOD monomers.

11. The polymer of claim 1, 2, 3, 4, 5 or 6 wherein the amino acid sequence of the spacer is substantially homologous to a connector domain or a fragment thereof, of a native endogenous protein which connector domain links functional domains of the endogenous protein in a manner that allows the functional domains to assume an appropriate tertiary structure and function independently.

12. The polymer of claim 11, wherein the connector domain is a hinge region of an immunoglobulin.

13. The polymer of claim 12 wherein the spacer has the amino acid sequence ProValProSerThrProProThrProSerProSerThrProProThrProSer.

14. A polymer of Cu/Zn superoxide dismutase of the formula
(SOD monomer-IgA-SOD monomer)$_x$ or
(SOD monomer-IgA-SOD monomer-IgA-SOD monomer)$_x$
wherein SOD monomer represents a Cu/Zn superoxide dismutase monomer, IgA represents a 10 to 100 amino acid long segment of an immunoglobulin hinge region, and x is an integer in the range of 2 and 4, inclusive.

15. A pharmaceutical composition for parenteral administration comprising the polymer of claim 1, 2, 3, 4, 5, 6, 7, 8, 9, 10, 11, 12, 13 or 14 admixed with a pharmaceutically acceptable vehicle that renders the composition injectable.

16. A method of increasing the circulatory life of Cu/Zn superoxide dismutase (SOD) comprising forming polymers of SOD units of at least two SOD monomers covalently coupled, carboxy terminus to amino terminus, to each other via a polypeptide spacer of at least three amino acids to make a polymer.

17. A composition of matter comprising at least two Cu/Zn superoxide dismutase (SOD) monomers covalently coupled, carboxy terminus to amino terminus, to each other by a polypeptide spacer of at least three amino acids.

18. DNA encoding a Cu/Zn superoxide dismutase (SOD) unit of at least two SOD monomers covalently coupled, carboxy terminus to amino terminus, to each other by a polypeptide spacer of at least three amino acids.

19. An expression vector for expressing the DNA of claim 18 in an organism comprising the DNA of claim 18 operably connected to DNA that enables the expression of the DNA in the organism.

20. A host organism containing the vector of claim 19 which permits the expression of the DNA encoding the SOD unit.

**Claims for the following Contracting States : ES, GR**

1. A method of increasing the circulatory life of Cu/Zn superoxide dismutase (SOD) comprising forming polymers of SOD units of at least two SOD monomers covalently coupled, carboxy terminus to amino terminus, to each other via a polypeptide spacer of at least three amino acids to make a polymer.

2. The method of claim 1 wherein the SOD in human SOD (hSOD).

3. The method of claim 2 wherein each hSOD unit has two hSOD monomers.

4. The method of claim 3 wherein the polymer contains 2 to 10 hSOD units.

5. The method of claim 3 wherein the polymer contains 2 to 4 hSOD units.

6. The method of claim 2 wherein the polymer contains 2 hSOD units.

7. The method of claim 1, 2, 3, 4, 5 or 6 wherein the spacer is 3 to 1000 amino acids in length, substantially nonimmunogenic, and substantially insensitive to proteases.

8. The method of claim 1, 2, 3, 4, 5 or 6 wherein the spacer is 10 to 100 amino acids in length, substantially nonimmunogenic, and substantially insensitive to proteases.

9. The method of claim 7 or 8 wherein the spacer lacks cysteine residues and is substantially nonhydrophobic.

10. The method of claim 2 wherein each hSOD unit has three hSOD monomers.

11. The method of claim 1, 2, 3, 4, 5 or 6 wherein the amino acid sequence of the spacer is substantially homologous to a connector domain or a fragment thereof, of a native endogenous protein which connector domain links functional domains of the endogenous protein in a manner that allows the functional domains to assume an appropriate tertiary structure and function independently.

12. The method of claim 11, wherein the connector domain is a hinge region of an immunoglobulin.

13. The method of claim 12 wherein the spacer has the amino acid sequence ProValProSerThrProProThrProSerProSerThrProProThrProSer.

14. A method according to claim 1 wherein the at least two SOD monomers are in the form of a polymer of Cu/Zn superoxide dismutase of the formula

(SOD monomer-IgA-SOD monomer)x or

(SOD monomer-IgA-SOD monomer-IgA-SOD monomer)x

wherein SOD monomer represents a Cu/Zn superoxide dismutase monomer, IgA represents a 10 to 100 amino acid long segment of an immunoglobulin hinge region, and x is an integer in the range of 2 and 4, inclusive.

15. A method according to any one of claims 1 to 14 which further comprises admixing the covalently coupled SOD monomers with a pharmaceutically acceptable vehicle.

16. The method claim 15 wherein the pharmaceutically acceptable vehicle renders the composition injectable.

17. A process for expression of DNA encoding a Cu/Zn superoxide dismutase (SOD) unit of at least two SOD monomers covalently coupled, carboxy terminus to amino terminus, to each other by a polypeptide spacer of at least three amino acids which comprises expressing a vector comprising said DNA in a host organism where the said DNA is operably connected to DNA that enables expression of said DNA in the organism.

## Patentansprüche

### Patentansprüche für folgende Vertragsstaaten : AT, BE, CH, DE, FR, GB, IT, LI, LU, NL, SE

1. Cu/Zn-Superoxid-Dismutase(SOD)-Polymeres aus SOD-Einheiten aus mindestens zwei SOD-Monomeren in kovalenter Kopplung, Carboxyl-Terminus an Amino-Terminus, aneinander durch einen Polypeptid-Spacer aus mindestens drei Aminosäuren.

2. Polymeres nach Anspruch 1, dadurch **gekennzeichnet** , daß die SOD Human-SOD (hSOD) ist.

3. Polymeres nach Anspruch 2, dadurch **gekennzeichnet** , daß jede hSOD-Einheit zwei hSOD-Monomere besitzt.

4. Polymeres nach Anspruch 3, dadurch **gekennzeichnet** , daß das Polymere zwei bis zehn hSOD-Einheiten enthält.

5. Polymeres nach Anspruch 3, dadurch **gekennzeichnet** , daß das Polymere zwei bis vier hSOD-Einheiten enthält.

6. Polymeres nach Anspruch 2, dadurch **gekennzeichnet** , daß das Polymere zwei hSOD-Einheiten enthält.

7. Polymeres nach Anspruch 1, 2, 3, 4, 5 oder 6, dadurch **gekennzeichnet** , daß der Spacer eine Länge von 3 bis 1000 Aminosäuren besitzt, im wesentlichen nicht immunogen und im wesentlichen unempfindlich gegenüber Proteasen ist.

8. Polymeres nach Anspruch 1, 2, 3, 4, 5 oder 6, dadurch **gekennzeichnet** , daß der Spacer eine Länge von 10 bis 100 Aminosäuren besitzt, im wesentlichen nicht immunogen und im wesentlichen unempfindlich gegenüber Proteasen ist.

9. Polymeres nach Anspruch 7 oder 8, dadurch **gekennzeichnet ,** daß der Spacer keine Cystein-Reste besitzt und im wesentlichen nicht hydrophob ist.

10. Polymeres nach Anspruch 2, dadurch **gekennzeichnet ,** daß jede hSOD-Einheit drei hSOD-Monomere besitzt.

11. Polymeres nach Anspruch 1, 2, 3, 4, 5 oder 6, dadurch **gekennzeichnet** , daß die Aminosäure-Sequenz des Spacers im wesentlichen einer Konnektor-Domäne oder einem Fragment davon eines nativen endogenen Proteins homolog ist, wobei die Konnektor-Domäne funktionelle Domänen des endogenen Proteins so verknüpft, daß die funktionellen Domänen eine geeignete Tertiärstruktur und Funktion unabhängig annehmen können.

12. Polymeres nach Anspruch 11, dadurch **gekennzeichnet ,**daß die Konnektor-Domäne eine Gelenk region eines Immunglobulins ist.

13. Polymeres nach Anspruch 12, dadurch **gekennzeichnet** , daß der Spacer die Aminosäuresequenz ProValProSerThrProProThrProSerProSerThrProProThrProSer besitzt.

14. Polymeres der Cu/Zn-Superoxid-Dismutase der Formel

(SOD-Monomeres-IgA-SOD-Monomeres)x oder

(SOD-Monomeres-IgA-SOD-Monomeres-IgA-SOD-Monomeres)x,

worin das SOD-Monomere für ein Cu/Zn-Superoxid-Dismutase Monomeres steht, IgA für ein 10 bis 100 Aminosäuren langes Segment einer Gelenkregion eines Immunglobulins steht, und x eine ganze Zahl im Bereich von 2 bis 4 einschließlich bedeutet.

15. Pharmazeutisches Präparat zur parenteralen Verabreichung, dadurch **gekennzeichnet** , daß es das Polymere nach einem der Anspruch 1, 2, 3, 4, 5, 6, 7, 8, 9, 10, 11, 12, 13 oder 14 im Gemisch mit einem pharmazeutisch annehmbaren Träger, der das Präparat injizierbar macht, enthält.

16. Verfahren zur Erhöhung der Kreislaufzeit der Cu/Zn-Superoxid-Dismutase (SOD), dadurch **gekennzeichnet**, daß man Polymere aus SOD-Einheiten aus mindestens zwei SOD-Monomeren in kovalenter Kopplung, Carboxyl-Terminus an Amino-Terminus, aneinander über einen Polypeptid-Spacer aus mindestens drei Aminosäuren bildet, um ein Polymeres herzustellen.

17. Präparat, dadurch **gekennzeichnet**, daß es mindestens zwei Cu/Zn-Superoxid-Dismutase (SOD)-Monomere in kovalenter Kopplung, Carboxyl-Terminus an Amino-Terminus, aneinander über einen Polypeptid-Spacer aus mindestens drei Aminosäuren umfaßt.

18. DNA, codierend für eine Cu/Zn-Superoxid-Dismutase (SOD) Einheit aus mindestens zwei SOD-Monomeren in kovalenter Kopplung, Carboxyl-Terminus an Amino-Terminus aneinander über einem Polypeptid-Spacer aus mindestens drei Aminosäuren.

19. Expressionsvektor zur Expression der DNA nach Anspruch 18 in einem Organismus, umfassend die DNA nach Anspruch 18 in ausführbarer Weise an DNA gebunden, die die Expression der DNA in dem Organismus ermöglicht.

20. Wirtsorganismus, umfassend den Vektor nach Anspruch 19, dadurch **gekennzeichnet**, daß er die Expression der DNA, die die SOD-Einheit codiert, gestattet.

**Patentansprüche für folgende Vertragsstaaten : ES, GR**

1. Verfahren zur Erhöhung der Kreislaufzeit der Cu/Zn-Superoxid-Dismutase (SOD), dadurch **gekennzeichnet**, daß man Polymere aus SOD-Einheiten aus mindestens zwei SOD-Monomeren in kovalenter Kopplung, Carboxyl-Terminus an Amino-Terminus, aneinander über einen Polypeptid-Spacer aus mindestens drei Aminosäuren bildet, um ein Polymeres herzustellen.

2. Verfahren nach Anspruch 1, dadurch **gekennzeichnet**, daß die SOD Human-SOD (hSOD) ist.

3. Verfahren nach Anspruch 2, dadurch **gekennzeichnet**, daß jede hSOD-Einheit zwei hSOD-Monomere besitzt.

4. Verfahren nach Anspruch 3, dadurch **gekennzeichnet**, daß das Polymere zwei bis zehn hSOD-Einheiten enthält.

5. Verfahren nach Anspruch 3, dadurch **gekennzeichnet**, daß das Polymere zwei bis vier hSOD-Einheiten enthält.

6. Verfahren nach Anspruch 2, dadurch **gekennzeichnet**, daß das Polymere zwei hSOD-Einheiten enthält.

7. Verfahren nach Anspruch 1, 2, 3, 4, 5 oder 6, dadurch **gekennzeichnet**, daß der Spacer eine Länge von 3 bis 1000 Aminosäuren besitzt, im wesentlichen nicht immunogen und im wesentlichen unempfindlich gegenüber Proteasen ist.

8. Verfahren nach Anspruch 1, 2, 3, 4, 5 oder 6, dadurch **gekennzeichnet**, daß der Spacer eine Länge von 10 bis 100 Aminosäuren besitzt, im wesentlichen nicht immunogen und im wesentlichen unempfindlich gegenüber Proteasen ist.

9. Verfahren nach Anspruch 7 oder 8, dadurch **gekennzeichnet**, daß der Spacer keine Cystein-Reste besitzt und im wesentlichen nicht hydrophob ist.

10. Verfahren nach Anspruch 2, dadurch **gekennzeichnet**, daß jede hSOD-Einheit drei hSOD-Monomere besitzt.

11. Verfahren nach Anspruch 1, 2, 3, 4, 5 oder 6, dadurch **gekennzeichnet**, daß die Aminosäure-Sequenz des Spacers im wesentlichen einer Konnektor-Domäne oder einem Fragment davon eines nativen endogenen Proteins homolog ist, wobei die Konnektor-Domäne funktionelle Domänen des endogenen Proteins so verknüpft, daß die funktionellen Domänen eine geeignete Tertiärstruktur und Funktion unabhängig annehmen können.

12. Verfahren nach Anspruch 11, dadurch **gekennzeichnet**, daß die Konnektor-Domäne eine Gelenkregion eines Immunglobulins ist.

13. Verfahren nach Anspruch 12, dadurch **gekennzeichnet**, daß der Spacer die Aminosäuresequenz ProValProSerThrProProThrProSerProSerThrProProThrProSer besitzt.

14. Verfahren nach Anspruch 1, dadurch **gekennzeichnet**, daß mindestens zwei SOD-Monomere in der Form eines Polymeren der Cu/Zn-Superoxid-Dismutase der Formel

(SOD-Monomeres-IgA-SOD-Monomeres)x oder

(SOD-Monomeres-IgA-SOD-Monomeres-IgA-SOD-Monomeres)x

vorliegen, worin das SOD-Monomere ein Cu/Zn-Superoxid-Dismutase-Monomeres bedeutet, IgA ein 10 bis 100 Aminosäuren langes Segment einer Gelenkregion eines Immunglobulins bedeutet, und x für eine ganze Zahl im Bereich von 2 bis 4 einschließlich steht.

15. Verfahren nach einem der Ansprüche 1 bis 14, dadurch **gekennzeichnet** , daß man weiterhin die kovalent gekoppelten SOD-Monomeren mit einem pharmazeutisch annehmbaren Träger mischt.

16. Verfahren nach Anspruch 15, dadurch **gekennzeichnet** , daß der pharmazeutisch annehmbare Träger das Präparat injizierbar macht.

17. Verfahren zur Expression einer DNA, die für eine Cu/Zn-Superoxid-Dismutase(SOD)-Einheit aus mindestens zwei SOD-Monomeren in kovalenter Kopplung, Carboxyl-Terminus an Amino-Terminus, aneinander durch einen Polypeptid-Spacer aus mindestens drei Aminosäuren codiert, dadurch **gekennzeichnet** , daß man einen Vektor, der die DNA umfaßt, in einem irtsorganismus, in dem die DNA in ausführbarer Weise an DNA, welche die Expression der DNA in dem Organismus ermöglicht, gekoppelt ist, exprimiert.


**Revendications**

**Revendications pour les Etats contractants suivant : AT, BE, CH, LI, DE, FR, GB, IT, LU, NL, SE**

1. Polymère de superoxyde dismutase (SOD) Cu/Zn à motifs SOD comprenant au moins deux monomères SOD couplés entre eux de façon covalente, par leurs groupements carboxy- et amino-terminaux respectivement, à l'aide d'un bras espaceur polypeptidique d'au moins trois acides aminés.

2. Polymère selon la revendication 1, dans lequel la SOD est la SOD humaine (hSOD).

3. Polymère selon la revendication 2, dans lequel chaque motif hSOD contient deux monomères hSOD.

4. Polymère selon la revendication 3, qui contient de 2 à 10 motifs hSOD.

5. Polymère selon la revendication 3, qui contient de 2 à 4 motifs hSOD.

6. Polymère selon la revendication 2, qui contient 2 motifs hSOD.

7. Polymère selon la revendication 1, 2, 3, 4, 5 ou 6, dand lequel le bras espaceur contient de 3 à 1000 acides aminés, est sensiblement non immunogène et est pratiquement insensible aux protéases.

8. Polymère selon la revendication 1, 2, 3, 4, 5 ou 6, dans lequel le bras espaceur contient 10 à 100 acides aminés, est sensiblement non immunogène et est pratiquement insensible aux protéases.

9. Polymère selon la revendication 7 ou 8, dans lequel le bras espaceur est exempt de restes cystéine et est sensiblement non hydrophobe.

10. Polymère selon la revendication 2, dans lequel chaque motif hSOD contient 3 monomères hSOD.

11. Polymère selon la revendication 1, 2, 3, 4, 5 ou 6 dans lequel la séquence d'acides aminés du bras espaceur est sensiblement homologue d'un domaine connecteur ou d'un fragment dudit domaine, d'une protéine native endogène, ledit domaine connecteur reliant des domaines fonctionnels de la protéine endogène de manière à permettre aux domaines fonctionnels d'adopter une structure tertiaire appropriée et de remplir leur fonction indépendamment.

12. Polymère selon la revendication 11, dans lequel le domaine connecteur est une région charnière d'une immonoglobuline.

13. Polymère selon la revendication 12, dans lequel le bras espaceur contient la séquence d'acides aminés suivante :

ProValProSerThrProProThrProSerProSerThrProProThrProSer.

14. Un polymère de superoxyde dismutase Cu/Zn de formule :

(monomère SOD-IgA-Monomère SOD)$_x$ ou

(monomère SOD-IgA-monomère SOD-IgA-monomère SOD)$_x$

dans laquelle monomère SOD représente un monomère superoxyde dismutase Cu/Zn, IgA représente un segment d'une région charnière d'une immunoglobuline, ayant de 10 à 100 acides aminés, et x est un entier dans la gamme de 2 à 4, bornes incluses.

15. Composition pharmaceutique pour l'administration par voie parentérale comprenant le polymère de la revendication 1, 2, 3, 4, 5, 6, 7, 8, 9, 10, 11, 12, 13 ou 14 en mélange avec un véhicule pharmaceutiquement acceptable compatible avec l'administration de la composition par injection.

16. Une méthode pour augmenter la durée de vie de la superoxyde dismutase (SOD) Cu/Zn circulante, comprenant l'étape consistant à préparer des polymères à motifs SOD comprenant au moins deux monomères SOD couplés entre eux, de façon covalente, par leurs groupements carboxy- et amino-terminaux respectivement à l'aide d'un bras espaceur polypeptidique ayant au moins trois acides aminés de façon à former un polymère.

17. Un produit comprenant au moins deux monomères de superoxyde dismutate (SOD) Cu/Zn couplés entre eux de façon covalente par leurs groupements carboxy- et amino-terminaux respectivement, à l'aide d'un bras espaceur polypeptidique ayant au moins trois acides aminés.

18. ADN codant pour un motif superoxyde dismutase (SOD) Cu/Zn comprenant au moins deux monomères SOD couplés entre eux de façon covalente, par leurs groupements carboxy- et amino-terminaux respectivement, à l'aide d'un bras espaceur polypeptidique ayant au moins trois acides aminés.

19. Un vecteur d'expression pour exprimer l'ADN de la revendication 18 dans un organisme comprenant l'ADN de la revendication 18 relié de façon fonctionnelle à l'ADN qui permet l'expression de l'ADN dans ledit organisme.

20. Un organisme hôte contenant le vecteur de la revendication 19 permettant l'expression de l'ADN codant pour ledit motif SOD.

**Revendications pour les Etats contractants suivant : ES, GR**

1. Une méthode pour augmenter la durée de vie de la superoxyde dismutase (SOD) Cu/Zn circulante, qui comprend l'étape consistant à former des polymères à motifs SOD comprenant au moins deux monomères SOD couplés entre eux de façon covalente, par leurs groupements carboxy- et amino-terminaux respectivement, à l'aide d'un bras espaceur polypeptidique ayant au moins trois acides aminés de façon à former un polymère.

2. La méthode de la revendication 1, dans laquelle la SOD est la SOD humaine (hSOD).

3. La méthode de la revendication 2, dans laquelle chaque motif hSOD contient deux monomères hSOD.

4. La méthode de la revendication 3, dans laquelle le polymère contient de 2 à 10 motifs hSOD.

5. La méthode de la revendication 3, dans laquelle le polymère contient de 2 à 4 motifs hSOD.

6. La méthode de la revendication 2, dans laquelle le polymère contient deux motifs hSOD.

7. La méthode de la revendication 1, 2, 3, 4, 5 ou 6, dans laquelle le bras espaceur contient de 3 à 1000 acides aminés, est sensiblement non immunogène et est pratiquement insensible aux protéases.

8. La méthode de la revendication 1, 2, 3, 4, 5 ou 6, dans laquelle le bras espaceur contient de 10 à 100 acides aminés, est sensiblement non immunogène et est pratiquement insensible aux protéases.

9. La méthode de la revendication 7 ou 8, dans laquelle le bras espaceur est exempt de restes cystéine et est sensiblement non hydrophobe.

10. La méthode de la revendication 2, dans laquelle chaque motif hSOD contient 3 monomères hSOD.

11. La méthode de la revendication 1, 2, 3, 4, 5 ou 6, dans laquelle la séquence d'acides aminés du bras espaceur est sensiblement homologue d'un domaine connecteur ou d'un fragment dudit domaine, d'une protéine native endogène, ledit domaine connecteur reliant des domaines fonctionnels de la protéine endogène de façon à permettre auxdits domaines fonctionnels d'adopter une structure tertiaire appropriée et de remplir leur fonction indépendamment.

12. La méthode de la revendication 11, dans laquelle le domaine connecteur est une région charnière d'une immunoglobuline.

13. La méthode de la revendication 12, dans laquelle le bras espaceur a la séquence d'acides aminés suivantes :

ProValProSerThrProProThrProSerProSerThrProProThrProSer.

14. Méthode selon la revendication 1, dans laquelle lesdits monomères SOD sont assemblés sous la forme d'un polymère de superoxyde dismutase Cu/Zn de formule :

(monomère SOD-IgA-monomère SOD)$_x$ ou

(monomère SOD-IgA-monomère SOD-IgA-monomère SOD)$_x$

dans laquelle monomère SOD représente un monomère de superoxyde dismutase Cu/Zn, IgA un fragment d'une région charnière d'une immunoglobuline, avant de 10 à 100 acides aminés et x est un entier dans la gamme de 2 à 4, bornes comprises.

15. Méthode selon l'une quelconque des revendications 1 à 14, qui comprend en outre le mélange des monomères SOD couplés de façon covalente, avec un véhicule pharmaceutiquement acceptable.

16. La méthode de la revendication 15, dans laquelle le véhicule pharmaceutiquement acceptable permet l'administration de la composition par injection.

17. Un procédé pour l'expression d'ADN codant pour un motif superoxyde dismutase (SOD) Cu/Zn comprenant au moins deux monomères SOD couplés entre eux de façon covalente, par leurs groupements carboxy- et amino-terminaux respectivement, à l'aide d'un bras espaceur polypeptidique ayant au moins trois acides aminés, qui comprend l'étape consistant à faire exprimer un vecteur comprenant ledit ADN dans un organisme hôte, ledit ADN étant lié de façon fonctionnelle à l'ADN qui permet l'expression dudit ADN dans ledit organisme.

EP 0 283 244 B1

CTGCAGCGTCTGGGGTTTCCGTTGCAGTCCTCGGAACCAGGACCTCGGCGTGGCCTAGC

```
                     1                                           10
          Met Ala Thr Lys Ala Val Cys Val Leu Lys Gly Asp Gly
GAGTT     ATG GCG ACG AAG GCC GTG TGC GTG CTG AAG GGC GAC GGC

                                         20
Pro Val Gln Gly Ile Ile Asn Phe Glu Gln Lys Glu Ser Asn Gly
CCA GTG CAG GGC ATC ATC AAT TTC GAG CAG AAG GAA AGT AAT GGA

          30                                       40
Pro Val Lys Val Trp Gly Ser Ile Lys Gly Leu Thr Glu Gly Leu
CCA GTG AAG GTG TGG GGA AGC ATT AAA GGA CTG ACT GAA GGC CTG

                                 50
His Gly Phe His Val His Glu Phe Gly Asp Asn Thr Ala Gly Cys
CAT GGA TTC CAT GTT CAT GAG TTT GGA GAT AAT ACA GCA GGC TGT

          60                                       70
Thr Ser Ala Gly Pro His Phe Asn Pro Leu Ser Arg Lys His Gly
ACC AGT GCA GGT CCT CAC TTT AAT CCT CTA TCC AGA AAA CAC GGT

                                 80
Gly Pro Lys Asp Glu Glu Arg His Val Gly Asp Leu Gly Asn Val
GGG CCA AAG GAT GAA GAG AGG CAT GTT GGA GAC TTG GGC AAT GTG

          90                                       100
Thr Ala Asp Lys Asp Gly Val Ala Asp Val Ser Ile Glu Asp Ser
ACT GCT GAC AAA GAT GGT GTG GCC GAT GTG TCT ATT GAA GAT TCT

                                 110
Val Ile Ser Leu Ser Gly Asp His Cys Ile Ile Gly Arg Thr Leu
GTG ATC TCA CTC TCA GGA GAC CAT TGC ATC ATT GGC CGC ACA CTG

          120                                      130
Val Val His Glu Lys Ala Asp Asp Leu Gly Lys Gly Gly Asn Glu
GTG GTC CAT GAA AAA GCA GAT GAC TTG GGC AAA GGT GGA AAT GAA

                                 140
Glu Ser Thr Lys Thr Gly Asn Ala Gly Ser Arg Leu Ala Cys Gly
GAA AGT ACA AAG ACA GGA AAC GCT GGA AGT CGT TTG GCT TGT GGT

          150           153
Val Ile Gly Ile Ala Gln OC
GTA ATT GGG ATC GCC CAA TAA
```

# FIG. 1

15

EP 0 283 244 B1

# CONSTRUCTION OF pCI/1SODHAI

## FIG. 2

The protein sequence shown in the synthetic DNA hinge:

IleAlaGlnProValProSerThrProProThrProSerProSerThrProProThrProSer

GATCGCCCAACCGGTACCATCTACCCCACCAACCCCATCTCCATCCACTCCACCAACTCCATC
CGGGTTGGCCATGGTAGATGGGGTGGTTGGGGTAGAGGTAGGTGAGGTGGTTGAGGTAGGTAC

16

## CONSTRUCTION OF pCI/I SOD HA-met

```
        |<        IgAI Hinge                                    >|<      Cu, Zn  HSOD                  >|
        KphI                                                                                            ApaI

             SerThrProProThrProSerProSerThrProProThrProSerAlaThrLysAlaValCysValLeuLysGlyAspGly
        5'   CATCTACCCCACCAACCCCATCTCCATCCACTCCACCAACTCCATCCGCTACAAAGGCTGTTTGTGTTTTGAAGGGTGACGGGCC  3'
        3'   CATGGTAGATGGGGTGGTTGGGGTAGAGGTAGGTGAGGTGGTTGAGGTAGGCGATGTTTCCGACAAACACAAAACTTCCCACTGC  5'
```

**FIG. 3**

EP 0 283 244 B1

EP 0 283 244 B1

DEAE Eluate

Heated Extract

Extract

— 43 KD

— 26 KD

FIG. 4

FIG. 5

FIG. 6

Fraction # →

hSOD  Input  81  72  66  60  57  51  MW

340 Kd
265 Kd  } High MW form
207 Kd

125 Kd trimer

} 75 Kd dimer

} 32 Kd monomer

## FIG. 7